(19) Europäisches Patentamt
European Patent Office
Office européen des brevets

(11) Publication number: **0 150 930**
**B1**

(12) **EUROPEAN PATENT SPECIFICATION**

(45) Date of publication of patent specification: **23.03.88**

(51) Int. Cl.$^4$: **C 07 C 59/305,** C 07 C 51/367
// C11D1/06, C23F11/12

(21) Application number: **85300164.2**

(22) Date of filing: **10.01.85**

(54) **Glycerine derivatives.**

(30) Priority: **31.01.84 US 575421**
**24.10.84 US 664451**

(43) Date of publication of application:
**07.08.85 Bulletin 85/32**

(45) Publication of the grant of the patent:
**23.03.88 Bulletin 88/12**

(84) Designated Contracting States:
**AT BE CH DE FR GB IT LI NL SE**

(56) References cited:
**DE-A-2 332 539**
**US-A-3 954 858**

**CHEMICAL ABSTRACTS, vol. 66, no. 2, 9th January 1967, page 328, no. 2818k, Columbus, Ohio, US; ZDENEK ORDELT: "Addition of 1,2-diols and glycidyl ethers to double bonds during polymerization with maleic anhydride. II. Addition of glycidyl butyl ether and reaction mechanisms" & CHEM. PRUMYSL 16(2), 97-100, (1966)**

(73) Proprietor: **A.E. STALEY MANUFACTURING COMPANY**
**2200 E Eldorado Street**
**Decatur Illinois 62521 (US)**

(72) Inventor: **Valenty, Vivian Briones**
**2631 Rosendale Road**
**Schenectady New York 12309 (US)**

(74) Representative: **Cockbain, Julian et al**
**Frank B. Dehn & Co. Imperial House 15-19, Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 150 930 B1

**Description**

Background of the invention

Field of the invention

This invention relates to glycerine derivatives useful as detergent builder materials and suitable as partial or total replacements for phosphates or nitrogen-containing builders.

Description of the art practices

Detergent builders are used to enhance the activity of the detergent or surfactant material used for cleaning. A detergent product typically contains a surface-active material (surfactant) which is used to lift dirt from the fabrics and to penetrate into the fabrics to remove embedded soil. Typically, these surface-active agents are the sodium salts of anionic materials. As the bulk of heavy-duty detergents are of the anionic nature, there exists an interference in the cleaning mechanism when calcium or magnesium ions (which are present as water hardness or body soil) react with the anion. In the case of body soil, the surfactant will become fixed onto the fabric due to the formation of the insoluble calcium or magnesium salt. The calcium or magnesium cations within the water cause the surfactant to be inactivated due to the formation of insoluble salts.

Heavy-duty liquid detergent products have been formulated but not successfully utilizing a detergent builder. This is most evident as the common detergent builders employed tend to be phosphate salts which precipitate out of a liquid composition when utilized at an effective amount for cleaning. Although heavy-duty liquid detergent products are often formulated with a substantial amount of a nonionic surfactant such as ethoxylated alcohol which is not metal ion sensitive, the ability of calcium and magnesium ions to fix on the soil leads to the desirable inclusion of a detergent builder.

As mentioned previously, phosphate salts (such as sodium tripolyphosphate or sodium pyrophosphate) have been extensively used. Several states in the U.S.A. have outlawed the use of phosphorus-containing compounds in detergent products due to the eutrification caused by the presence of the phosphates. Replacements for phosphates as builders in detergent products have included organic nitrogen-containing compounds, carbonates and aluminosilicates. Each of these materials has its own particular negatives associated therewith. For instance, concern has been expressed over the wide-spread usage of organic nitrogen-containing compounds due to potential carcinogenic effects in the water supply. Carbonates have been widely employed but are generally ineffective as detergent builders as they result in the build-up of scale due to insoluble calcium carbonate formation. Aluminosilicates are insoluble materials commonly used in water softeners. The aluminosilicates are disadvantageous in that, as an insoluble material, they can foul sewer lines and water-treatment facilities if used in excessive amounts. The aluminosilicates are also not useful in liquid products due to their insolubility.

It is, therefore, desirable to formulate detergent products containing builders which do not contain nitrogen or phosphorus and which are water-soluble and are biodegradable. It has been reported in an article entitled "Nitrogen- and Phosphorus-Free Strong Sequestering Building", Kemper et al. *Tenside Detergents, 12* page 47—51 (1975) that the reaction product of ethylene glycol and dimethyl diazomalonate results in such a compound. While this material avoids the presence of phosphorus or nitrogen in the desired compound, it does allow for the potential presence of the diazo compound in the environment and requires exaggerated temperature and the use of copper as a catalyst to form the desired compound. The efficacy of this compound as reported by the authors is rated at about 97% of sodium tripolyphosphate. Other compounds disclosed in the Kemper reference show Builder M (2 - oxa - 1,1,3 - propanetricarboxylic acid) at 93% of sodium tripolyphosphate. A further proposed material 2 - oxa - 1,3,4 - butanetricarboxylic acid (CMOS) is rated at only 90% of sodium tripolyphosphate.

The background to this art is further described in United States Patent 3,692,685, issued to Lamberti et al on September 19, 1972, as well as United States Patent 3,128,287, issued to Berg on April 7, 1964. Further disclosures of carboxylic acid materials are found in United States Patent 4,021,376 issued to Lamberti et al on May 3, 1977.

German Patent 2,147,780, published on March 29, 1973, to Kandler et al and German Patent 2,408,591, published on September 4, 1975, to Borggrefe et al, also concern the general subject matter of the present invention. The article of Crutchfield entitled "Organic Builders: A Review of Worldwide Efforts to Find Organic Replacements for Detergent Phosphates" published in the *JAOCS 55*, pages 58—65 (1978), and the Matzner et al article entitled "Organic Builder Salts as Replacements for Sodium Tripolyphosphate (1) "in *Tenside Detergents, 10*, pages 119—125 and 239—245 (1973) also provide useful information concerning the general scope of the present invention. Further information on detergent products is found in Stubbs et al United States Patent 4,017,541 issued April 12, 1977 and Borggrefe's United States Patents 4,002,676 and 4,219,672 issued January 11, 1977 and August 26, 1980, respectively.

Summary of the invention

It is an objective of the present invention to provide compounds suitable for use as detergent builders and producible from readily available materials which do not contain phosphorus or nitrogen.

Thus, in one aspect, the invention provides compounds of formula A

$$R^1O—CH_2—CH(OR^2)—CH_2—OR^3 \qquad (A)$$

(wherein $R^1$, $R^2$ and $R^3$, which may be the same or different, each represents a hydrogen atom or a group of formula —CH(COOH)—$CH_2$—COOH, with the proviso that at least one of $R^1$, $R^2$ and $R^3$ represents other than a hydrogen atom), salts thereof and mixtures of two or more said compounds or salts.

More specifically, the invention provides compounds of formulae

$$[MOOCCH_2(MOOC)CHOCH_2]_2CHOCH(COOM)CH_2COOM, \quad (I)$$

$$HOCH_2CH(OH)CH_2OCH(COOM)CH_2COOM, \quad (II)$$

$$HOCH_2CH[OCH(COOM)CH_2COOM]CH_2OCH(COOM)CH_2COOM, \quad (III)$$

$$MOOCCH_2(MOOC)CHOCH_2CH(OH)CH_2OCH(COOM)CH_2COOM, \quad (IV)$$

and

$$MOCH_2CH[OCH(COOM)CH_2COOM]CH_2OH \quad (V)$$

(wherein each M, which may be the same or different, represents a salt-forming cation or hydrogen) and mixtures thereof.

The compounds of the invention may be prepared by the reaction of glycerine with source of maleic acid, such as maleic anhydride, in the presence of a base.

Thus, in a further aspect, the invention provides a process for the preparation of glycerine derivatives which process comprises reacting glycerine with a source of maleic acid in the presence of an alkaline earth metal hydroxide.

The compounds of the invention are biodegradable, readily soluble in water and have superior capabilities as detergent builders over known related compounds. The compounds may be used in the cleaning of fabrics there to control calcium, magnesium and iron ions during the cleaning process.

Detailed description of the invention

The compounds and salts of the present invention are glycerine derivatives. Glycerine is a trihydroxylic compound containing two primary hydroxyl groups and one centrally located secondary hydroxyl group. The positioning of the secondary hydroxyl group makes it less likely to react to give the desired compounds of the present invention. The primary compounds obtained herein are the trisubstituted product (Compound I) and the 1,3 disubstituted product (Compound IV). The secondary materials obtained herein are the 1,2-disubstituted material (Compound III); the 1 (being equivalent with the 3 position) substituted mono adduct (Compound II) and the 2 substituted mono adduct (Compound V). Glycerine is a readily available material and any source may be utilized to obtain the compounds described herein.

The second reactant material in the process for producing the compounds of the present invention is preferably maleic anhydride. Maleic acid may also be used, however, maleic anhydride is less expensive and readily available. If it is desired to conduct the reaction through maleic acid, the maleic anhydride may be simply converted through the addition of water to give the corresponding acid. The term source of maleic acid is defined to mean a material which will generate maleic acid and which is useable to give the products of the present invention.

An alkaline earth metal catalyst, preferably calcium hydroxide, is utilized in the formation of the desired compounds. As the hydroxide acts both as a catalyst and as a preferred agent for keeping the pH within the desired range, it will be added as necessary to maintain the pH within the desired range. The pH of the reaction mixture is preferably greater than 10 and less than 14, with a desirable range being from about 10.5 to 12.5. The pH may be further adjusted with caustic. At the end of the reaction time, the alkaline earth ions (e.g. calcium or magnesium from the respective hydroxides) may be removed by precipitation with a soluble carbonate salt such as sodium carbonate or sodium bicarbonate. This precipitate may then be filtered off leaving the sodium salt of the compounds of the invention in the aqueous solution. Similarly, the potassium salt may be obtained as well as the ammonium and substituted ammonium salts. If desired the alkaline earth metal salt of compounds (I—V) may be recovered directly from the reaction mixture.

The temperature during the formation of the compounds of the present invention is conveniently maintained in the reactor at greater than 50°C, typically from 50°C to 125°C, preferably 60°C to 120°C.

The crude product may be used as a builder or may be conveniently isolated as the sodium salt. The reaction mixture is then acidified to a pH of about 2 with an acid such as concentrated hydrochloric for purification followed by the addition of caustic. The addition of the acid will result in the precipitation of fumaric acid which is then filtered from the reaction mixture. The filtrate may be evaporated to dryness in a rotary evaporator with the residue extracted with an excess of 2-butanol to separate the product from inorganic salts. This product is then filtered and the filtrate is evaporated to dryness. The product is then ready for use in its intended purpose.

In another aspect, the invention provides a chemical composition comprising at least one compound of formula A or salt thereof in admixture with at least one carrier material or chemically active agent, such as a surfactant or a further detergent builder.

The compounds of the present invention are conveniently used as detergent builders in formulations

with surfactants, for example alkyl ether sulfates, alkyl benzene sulfonates, alkyl sulfates, olefin sulfonates, paraffin sulfonates, alkoxylated alcohols (especially ethoxylated alcohols) and alkyl polyglycosides and mixtures thereof. Conveniently, the novel detergent builders of the present invention are utilized in a weight ratio of from about 4:1 to about 1:4, preferably 3:1 to 1:3 by weight to the surfactant. The detergent products which may be formulated according to the present invention may suitably be liquid and conveniently are used at from about 0.05 to 1% by weight of the wash liquor, e.g. water.

Detergent products formulated according to the present invention may also include a co-builder such as carboxymethyloxysuccinate; (2 - oxa - 1,3,4 - butanetricarboxylic acid); Builder M (2 - oxa - 1,1,3 - propane - tricarboxylic acid); zeolites including the type referred to in United States Patent 4,019,999 issued April 26, 1977 to Ohren et al. Similarly, citrates, carbonates and various phosphates including tripolyphosphate, pyrophosphates, and orthophosphates may be utilized as co-builders. The phosphate materials and a material such as the salts of nitrilotriacetic acid which may be used are, for the foregoing reasons, undesirable due to their environmental consequences. Nonetheless, should specific uses be desired, such materials may be utilized. The builders (I), (II), (III), (IV) and (V) are conveniently used in a weight ratio of 8:1 to 1:8 with each other. Particularly effective are mixtures of (I) with (III) and/or (IV). Mixtures of (I) and (II) as well as (I) through (V) are also useful.

Other convenient materials which may be utilized in formulating detergent products include sodium sulfate which is typically used as a structurant in a detergent product and sodium silicate which is useful as a structurant in granular detergent products and as well to protect washing machine surfaces from corrosion. Silicates also function to control pH in the wash liquor. Anti-caking agents for granular products, and hydrotropes and viscosity agents may be included for liquid products. Optical dyes and brighteners are also useful in combination with the builders of this invention.

In a still further aspect, the invention provides the use of compounds of formula A, salts thereof and compositions thereof in or as detergent products.

In another aspect the invention provides the use of salts of compounds of formula A as corrosion inhibitors. Thus in particular such salts may be used as a leached corrosion inhibitor such as by pumping the product into a well-hole and allowing it to slowly solubilize thereby protecting the piping in the well-hole from corrosion.

Throughout the specification and claims, percentages and ratios are by weight, and temperatures are in degrees Celsius unless otherwise indicated.

The following non-limiting Examples are provided to illustrate the present invention:

Example I

A mixture is prepared containing three moles of glycerin, 5.25 moles of calcium hydroxide, 11.55 moles caustic, and 42 moles of water. The reaction is initiated by adding 10.5 moles of maleic anhydride. The mixture is vigorously stirred and the temperature is maintained at about 90°C during addition of the maleic anhydride. The pH is maintained between 11 and 12 and the reaction is allowed to continue at reflux for about 2.5 hours.

The reaction is now essentially complete and the reaction mixture is allowed to cool. At this time about 5.51 moles of sodium carbonate in 125 moles water are added with vigorous stirring. The addition of sodium carbonate causes calcium present in the reaction mixture to precipitate. The calcium carbonate is then filtered off after the reaction has been cooled to ambient temperature. The filtrate contains components of formulae (I) to (V). Compound (I) of the present invention is determined to be more effective on a molar basis than sodium tripolyphosphate or NTA in its ability to rapidly sequester calcium ions from solution.

Example II

A mixture of 0.45 parts of linear dodecyl benzene sulfonate and 0.25 parts of the builder material of Example I or of a prior art builder (sodium tripolyphosphate) are added to 1000 parts of water containing calcium and magnesium for a total hardness level of 200 ppm as calcium carbonate in a 3:2 calcium to magnesium ratio. The mixture is adjusted to pH 9.0 and transferred to a tergotometer bucket which is preheated to 40.5°C. After agitation for 30 seconds, 6 soiled cloth (dacron/cotton blend) swatches with known reflectance values are added to each bucket. Agitation is continued at 125 rpm for 15 minutes. The cloth swatches are rinsed in water at 37°C for 2 minutes and then dried in a clothes dryer for 15 minutes. The dried swatches are ironed before determination of the change in reflectance is made. The results show the builder of Example I to be effective.

A second test at a wash temperature of 49°C gives similar results. At an equivalent weight level, the present builder outperforms sodium tripolyphosphate in calcium control. Products formulated as above give excellent hot or cold water cleaning ability.

Example III

A product according to the present invention is prepared utilizing 20 parts builder prepared by Example I, 12 parts of the triethoxylated alcohol (dodecyl) and 50 parts water. The detergent product so formulated is fully miscible and shelf-stable, i.e. without separation of the components. The product, when tested, performs superior to a similarly formulated unbuilt detergent composition.

Example IV

Products of the present invention are prepared by reacting 19.7 parts of maleic anhydride in 100 parts water for about 15 minutes. 55.1 parts of glycerine are added followed by 27 parts of calcium hydroxide. The mixture is vigorously stirred and the temperature is raised to 100°C. The pH is maintained between 11 and 12.

After about one-half hour at 100°C, the mixture is cooled to 60—75°C by means of an air stream and five portions of 19 parts of maleic anhydride are added over time to the reaction mixture. A last portion of 58.8 parts of maleic anhydride is then added. The maleic anhydride is previously combined with 5 parts of water per part maleic anhydride prior to the addition to the reaction mixture. Each addition of maleic anhydride is followed by an equimolar addition of calcium hydroxide to maintain the pH at 11 to 12. The reaction is allowed to continue at 100°C for an additional 3 to 3.5 hours.

The reaction is now essentially complete and the reaction mixture is allowed to cool to about 80—90°C. At this time about 250 parts of sodium carbonate and sufficient water to keep the mixture fluid are added with vigorous stirring. The addition of sodium carbonate causes the calcium present in the reaction mixture to precipitate. The calcium carbonate is then filtered off after the reaction has been cooled to ambient temperature. The filtrate contains components of formulae (I) to (V). The products of the present invention are determined to be more effective than sodium tripolyphosphate in their ability to sequester calcium ions from solution.

Example V

A mixture of 0.45 parts of linear dodecyl benzene sulphonate and 0.25 parts of the builder listed below are added to 1000 parts of water containing calcium and magnesium for a total hardness level of 200 ppm as calcium carbonate in a 3:2 calcium to magnesium ratio. The mixture is adjusted to pH 9.0 and transferred to a tergotometer bucket which is preheated to 40.5°C. After agitation for 30 seconds, 6 soiled cloth (dacron/cotton blend) swatches with known reflectance values are added to each bucket. Agitation is continued at 125 rpm for 15 minutes. The cloth swatches are rinsed in water at 37°C for 2 minutes and then dried in a clothes dryer for 15 minutes. The dried swatches are ironed before determination of the change in reflectance is made. The results are as follows:

| Run | Builder | Reflectance values |
|---|---|---|
| 1 | Sodium tripolyphosphate | 143 |
| 2 | Material of Example IV | 173 |

A second test at a wash temperature of 49°C gives similar results. At an equivalent weight level, the builder Example IV outperforms sodium tripolyphosphate in calcium control. Products formulated as above give excellent hot or cold water cleaning ability.

Example VI

A product according to the present invention is prepared utilizing 20 parts builder prepared by Example IV, 12 parts of the triethoxylated alcohol (dodecyl) and 50 parts water. The detergent product so formulated is fully miscible and shelf-stable, i.e. without separation of the components. The product, when tested, performs superior to a similarly formulated unbuilt detergent composition.

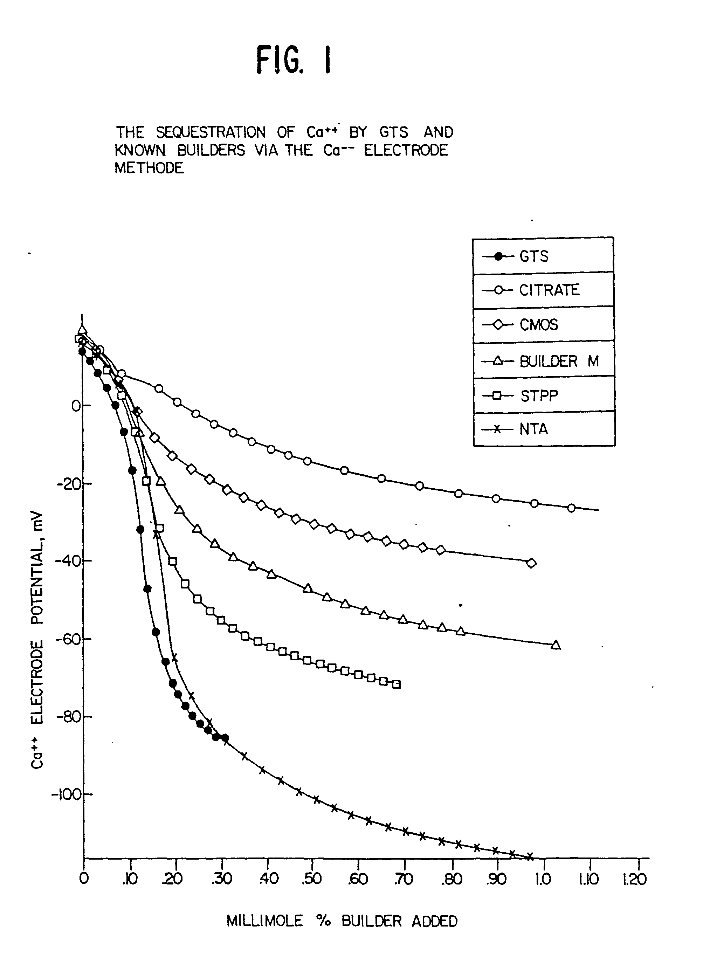

The advantageous nature of the compounds of the present invention is further illustrated by the accompanying drawing, Fig. 1, which shows the molar concentration of selected builders versus calcium ion electrode potential.

The proximity of the curve to the Y-axis shows the speed with which the builder acts to sequester free calcium ion. Proximity of the curve to the X-axis shows the ability to control calcium ion to low levels in the solution. The latter point is helpful in cleaning as the residual free calcium level is still capable of binding soil to fabrics.

GTS (Compound I) is superior to all other builders in its ability to rapidly control calcium ions. The other builders in the test (all sodium salts) were nitrilotriacetate (NTA): tripolyphosphate (STPP); CMOS; citrate; and Builder M.

**Claims for the Contracting States: BE, CH, DE, FR, GB, IT, LI, NL, SE**

1. Compounds of formula A

$$R^1O—CH_2—CH(OR^2)—CH_2—OR^3 \quad (A)$$

(wherein $R^1$, $R^2$ and $R^3$, which may be the same or different, each represents a hydrogen atom or a group of formula $—CH(COOH)—CH_2—COOH$, with the proviso that at least one of $R^1$, $R^2$ and $R^3$ represents other than a hydrogen atom), salts thereof and mixtures of two or more said compounds or salts.

2. Compounds as claimed in claim 1 of formula I

$$[MOOC—CH_2—CH(COOM)—O—CH_2—]_2CH—O—CH(COOM)—CH_2—COOM \qquad (I)$$

(wherein each M, which may be the same or different, represents a hydrogen atom or a salt-forming cation) and mixtures thereof.

3. Compounds as claimed in claim 1 of formula II

$$HO—CH_2—CH(OH)—CH_2—O—CH(COOM)—CH_2—COOM \qquad (II)$$

(wherein each M, which may be the same or different, represents a hydrogen atom or a salt-forming cation) and mixtures thereof.

4. Compounds as claimed in claim 1 of formula III

$$HO—CH_2—CH—CH_2—O—CH(COOM)—CH_2—COOM \qquad (III)$$
$$|$$
$$O—CH(COOM)—CH_2—COOM$$

(wherein each M, which may be the same or different, represents a hydrogen atom or a salt-forming cation) and mixtures thereof.

5. Compounds as claimed in claim 1 of formula IV

$$MOOC—CH_2—CH(COOM)—O—CH_2—CH(OH)—CH_2—O—CH(COOM)—CH_2—COOM \qquad (IV)$$

(wherein each M, which may be the same or different, represents a hydrogen atom or a salt-forming cation) and mixtures thereof.

6. Compounds as claimed in claim 1 of formula V

$$HO—CH_2—CH—CH_2—OH \qquad (V)$$
$$|$$
$$O—CH(COOM)—CH_2—COOM$$

(wherein each M, which may be the same or different, represents a hydrogen atom or a salt-forming cation) and mixtures thereof.

7. Compounds as claimed in any one of claims 1 to 6 in the form of the free acids.

8. Compounds as claimed in any one of claims 1 to 6 in salt form, the counter cation being selected from sodium, potassium, calcium, magnesium and optionally substituted ammonium ions.

9. A mixture as claimed in claim 1 of compounds of formula I (as defined in claim 2) and compounds of formula III (as defined in claim 4).

10. A mixture as claimed in claim 1 of compounds of formula I (as defined in claim 2) and compounds of formula IV (as defined in claim 5).

11. A mixture as claimed in either of claims 9 and 10 wherein said compounds are in a weight ratio of from 8:1 to 1:8.

12. A chemical composition comprising at least one compound of formula A or salt thereof as claimed in any one of claims 1 to 11 in admixture with at least one carrier material or chemically active agent.

13. A composition as claimed in claim 12 comprising as a said chemically active agent a surfactant.

14. A composition as claimed in claim 13 wherein said surfactant is selected from alkyl ether sulfates, alkyl benzene sulfonates, alkyl sulfates, olefin sulfonates, paraffin sulfonates, alkyl polyglycosides, alkoxylated alcohols, and mixtures of two or more thereof.

15. A composition as claimed in any one of claims 12 to 14 comprising as a said chemically active agent a detergent builder selected from 2 - oxa - 1,3,4 - butanetricarboxylic acid salts, 2 - oxa - 1,1,3 - propanetricarboxylic acid salts, zeolites, citrate salts, carbonate salts, silicates, phosphate salts and nitrilotriacetic acid salts.

16. A composition as claimed in any one of claims 12 to 15 in the form of a liquid detergent product.

17. The use of compounds, salts and compositions as claimed in any one of claims 1 to 16 in or as detergent products.

18. The use of salts as claimed in any one of claims 1 to 11 as corrosion inhibitors.

19. A process for the preparation of glycerine derivatives which process comprises reacting glycerine with a source of maleic acid in the presence of an alkaline earth metal hydroxide.

20. A process as claimed in claim 19 wherein as said source of maleic acid is used maleic anhydride.

21. Glycerine derivatives produced by a process as claimed in either of claims 19 and 20.

22. A process for the preparation of chemical compositions as claimed in any one of claims 12 to 16 which process comprises admixing said compounds of formula A or salts thereof with said carrier material and/or said chemically active agent.

**Claims for the Contracting State: AT**

1. A process for the preparation of glycerine derivatives which process comprises reacting glycerine with a source of maleic acid in the presence of an alkaline earth metal hydroxide.

2. A process as claimed in claim 1 being a process for the preparation of compounds of formula A

$$R^1O—CH_2—CH(OR^2)—CH_2—OR^3 \qquad (A)$$

(wherein $R^1$, $R^2$ and $R^3$, which may be the same or different, each represents a hydrogen atom or a group of formula —CH(COOH)—$CH_2$—COOH, with the proviso that at least one of $R^1$, $R^2$ and $R^3$ represents other than a hydrogen atom) or salts or mixtures thereof.

3. A process as claimed in claim 2 being a process for the preparation of compounds of formula I

$$[MOOC—CH_2—CH(COOM)—O—CH_2—]_2CH—O—CH(COOM)—CH_2—COOM \qquad (I)$$

(wherein each M, which may be the same or different, represents a hydrogen atom or a salt-forming cation) or mixtures thereof.

4. A process as claimed in claim 2 being a process for the preparation of compounds of formula II

$$HO—CH_2—CH(OH)—CH_2—O—CH(COOM)—CH_2—COOM \qquad (II)$$

(wherein each M, which may be the same or different, represents a hydrogen atom or a salt-forming cation) or mixtures thereof.

5. A process as claimed in claim 2 being a process for the preparation of compounds of formula III

$$\begin{array}{l} HO—CH_2—CH—CH_2—O—CH(COOM)—CH_2—COOM \qquad (III) \\ \qquad\qquad\quad | \\ \qquad\qquad\quad O—CH(COOM)—CH_2—COOM \end{array}$$

(wherein each M, which may be the same or different, represents a hydrogen atom or a salt-forming cation) or mixtures thereof.

6. A process as claimed in claim 2 being a process for the preparation of compounds of formula IV

$$MOOC—CH_2—CH(COOM)—O—CH_2—CH(OH)—CH_2—O—CH(COOM)—CH_2—COOM \qquad (IV)$$

(wherein each M, which may be the same or different, represents a hydrogen atom or a salt-forming cation) or mixtures thereof.

7. A process as claimed in claim 2 being a process for the preparation of compounds of formula V

$$\begin{array}{l} HO—CH_2—CH—CH_2—OH \qquad (V) \\ \qquad\qquad\quad | \\ \qquad\qquad\quad O—CH(COOM)—CH_2—COOM \end{array}$$

(wherein each M, which may be the same or different, represents a hydrogen atom or a salt-forming cation) or mixtures thereof.

8. A process as claimed in any one of claims 1 to 7 wherein as said source of maleic acid is used maleic anhydride.

9. A process as claimed in any one of claims 1 to 8 wherein as said alkaline earth metal hydroxide is used calcium hydroxide.

10. A process as claimed in any one of claims 1 to 9 wherein the reaction is effected at a pH of greater than 10 and less than 14.

11. A process as claimed in any one of claims 1 to 10 wherein the reaction is effected at a pH of 10.5 to 12.5.

12. A process as claimed in any one of claims 1 to 11 wherein the reaction is effected at a temperature of greater than 50°C.

13. A process as claimed in any one of claims 1 to 12 wherein subsequent to the reaction between glycerine and said source of maleic acid alkaline earth metal ions are removed by precipitation using a soluble carbonate salt.

14. A process as claimed in any one of claims 1 to 13 comprising sequential addition to a glycerine-containing reaction mixture of portions of maleic anhydride and of calcium hydroxide.

15. A chemical composition comprising at least one compound of formula A (as defined in claim 2) or salt thereof in admixture with at least one carrier material or chemically active agent.

16. A composition as claimed in claim 15 comprising as a said chemically active agent a surfactant.

17. A composition as claimed in claim 16 wherein said surfactant is selected from alkyl ether sulfates,

alkyl benzene sulfonates, alkyl sulfates, olefin sulfonates, paraffin sulfonates, alkyl polyglycosides, alkoxylated alcohols, and mixtures of two or more thereof.

18. A composition as claimed in any one of claims 15 to 17 comprising as a said chemically active agent a detergent builder selected from 2 - oxa - 1,3,4 - butanetricarboxylic acid salts, 2 - oxa - 1,1,3 - propanetricarboxylic acid salts, zeolites, citrate salts, carbonate salts, silicates, phosphate salts and nitrilotriacetic acid salts.

19. A composition as claimed in any one of claims 15 to 18 in the form of a liquid detergent product.

20. The use of compounds of formula A (as defined in claim 2) or salts thereof in or as detergent products.

21. The use of compounds of formula A (as defined in claim 2) or salts thereof as corrosion inhibitors.

22. A process for the preparation of chemical compositions as claimed in any one of claims 15 to 19 which process comprises admixing compounds of formula A (as defined in claim 2) or salts thereof with a carrier material and/or a chemically active agent.

**Patentansprüche für die Vertragsstaaten: BE, CH, DE, FR, GB, IT, LI, NL, SE**

1. Verbindungen von Formel (A):

$$R^1O—CH_2—CH(OR^2)—CH_2—OR^3 \qquad (A)$$

(worin $R^1$, $R^2$ und $R^3$, die gleich oder verschieden sein können, jeweils ein Wasserstoffatom oder eine Gruppe der Formel $—CH(COOH)—CH_2—COOH$ bedeuten, unter der Voraussetzung, daß wenigstens einer der Reste $R^1$, $R^2$ oder $R^3$ kein Wasserstoffatom ist), Salze davon und Mischungen von zwei oder mehreren der Verbindungen oder Salze.

2. Verbindungen nach Anspruch 1 der Formel (I):

$$[MOOC—CH_2—CH(COOM)—O—CH_2—]_2CH—O—CH(COOM)—CH_2—COOM \qquad (I)$$

worin die Reste M, die gleich oder verschieden sein können, jeweils ein Wasserstoffatom oder ein salzbildendes Kation bedeuten), und Mischungen davon.

3. Verbindungen nach Anspruch 1 der Formel (II):

$$HO—CH_2—CH(OH)—CH_2—O—CH(COOM)—CH_2—COOM \qquad (II)$$

worin die Reste M, die gleich oder verschieden sein können, jeweils ein Wasserstoffatom oder ein salzbildendes Kation bedeuten), und Mischungen davon.

4. Verbindungen nach Anspruch 1 der Formel (III):

$$\begin{array}{l} HO—CH_2—\underset{|}{C}H—CH_2—O—CH(COOM)—CH_2—COOM \\ \qquad\quad\;\; O—CH(COOM)—CH_2—COOM \end{array} \qquad (III)$$

worin die Reste M, die gleich oder verschieden sein können, jeweils ein Wasserstoffatom oder ein salzbildendes Kation bedeuten), und Mischungen davon.

5. Verbindungen nach Anspruch 1 der Formel (IV):

$$MOOC—CH_2—CH(COOM)—O—CH_2—CH(OH)—CH_2—O—CH(COOM)—CH_2—COOM \qquad (IV)$$

worin die Reste M, die gleich oder verschieden sein können, jeweils ein Wasserstoffatom oder ein salzbildendes Kation bedeuten), und Mischungen davon.

6. Verbindungen nach Anspruch 1 der Formel (V):

$$\begin{array}{l} HO—CH_2—\underset{|}{C}H—CH_2—OH \\ \qquad\quad\;\; O—CH(COOM)—CH_2—COOM \end{array} \qquad (V)$$

worin die Reste M, die gleich oder verschieden sein können, jeweils ein Wasserstoffatom oder ein salzbildendes Kation bedeuten), und Mischungen davon.

7. Verbindungen nach einem der Ansprüche 1 bis 6 als freie Säuren.

8. Verbindungen nach einem der Ansprüche 1 bis 6 als Salze, wobei das Gegenkation ausgewählt ist unter Natrium-, Kalium-, Calcium-, Magnesium- und gegebenenfalls substituierten Ammoniumionen.

9. Mischung nach Anspruch 1 von Verbindungen der Formel (I) (wie in Anspruch 2 angegeben), und Verbindungen von Formel (III) (wie in Anspruch 4 angegeben).

10. Mischung nach Anspruch 1 von Verbindungen der Formel (I) (wie in Anspruch 2 angegeben), und Verbindungen von Formel (IV) (wie in Anspruch 5 angegeben).

11. Mischung nach Anspruch 9 oder 10, worin die Verbindungen in einem Gewichtsverhältnis von 8:1 bis 1:8 vorliegen.

12. Chemisches Mittel, das wenigstens eine Verbindung der Formel (A) oder ein Salz davon, nach einem der Ansprüche 1 bis 11, zusammen mit wenigstens einem Trägermaterial oder chemisch aktiven Agens enthält.

13. Mittel nach Anspruch 12, das als chemisch aktives Agens ein oberflächenaktives Agens enthält.

14. Mittel nach Anspruch 13, wobei das oberflächenaktive Agens ausgewählt ist unter Alkylethersulfaten, Alkylbenzolsulfonaten, Alkylsulfaten, Olefinsulfonaten, Paraffinsulfonaten, Alkylpolyglycosiden, alkoxylierten Alkoholen und Mischungen von zwei oder mehreren davon.

15. Mittel nach einem der Ansprüche 12 bis 14, das als chemisch aktives Agens einen Detergensbuilder enthält, ausgewählt unter 2 - Oxa - 1,3,4 - butantricarbonsäuresalzen, 2 - Oxa - 1,1,3 - propantricarbonsäuresalzen, Zeoliten, Citratsalzen, Carbonatsalzen, Silikaten, Phosphatsalzen und Nitrilotriessigsäuresalzen.

16. Mittel nach einem der Ansprüche 12 bis 15 als flüssiges Detergensprodukt.

17. Verwendung von Verbindungen, Salzen und Mitteln nach einem der Ansprüche 1 bis 16 in oder als Detergensprodukt(en).

18. Verwendung von Salzen nach einem der Ansprüche 1 bis 11 als Korrosionsinhibitoren.

19. Verfahren zur Herstellung von Glyzerinderivaten, wobei man Glyzerin mit einer Quelle für Maleinsäure in Anwesenheit eines Erdalkalimetallhydroxids umsetzt.

20. Verfahren nach Anspruch 19, wobei man als Quelle für Maleinsäure Maleinsäureanhydrid verwendet.

21. Glyzerinderivate, die gemäß dem Verfahren nach Anspruch 19 oder 20 hergestellt sind.

22. Verfahren zur Herstellung von chemischen Mitteln nach einem der Ansprüche 12 bis 16, wobei man die Verbindungen der Formel (A) oder Salze davon mit dem Trägermaterial und/oder chemisch aktiven Agens vermischt.

**Patentansprüche für den Vertragsstaat: AT**

1. Verfahren zur Herstellung von Glyzerinderivaten, wobei man Glyzerin mit einer Quelle für Maleinsäure in Anwesenheit eines Erdalkalimetallhydroxids umsetzt.

2. Verfahren nach Anspruch 1, wobei man Verbindungen der Formel (A):

$$R^1O—CH_2—CH(OR^2)—CH_2—OR^3 \qquad (A)$$

(worin $R^1$, $R^2$ und $R^3$, die gleich oder verschieden sein können, jeweils ein Wasserstoffatom oder eine Gruppe der Formel $—CH(COOH)—CH_2—COOH$ bedeuten, unter der Voraussetzung, daß wenigstens einer der Reste $R^1$, $R^2$ oder $R^3$ kein Wasserstoffatom ist), oder Salze oder Mischungen davon herstellt.

3. Verfahren nach Anspruch 2, wobei man Verbindungen von Formel (I):

$$[MOOC—CH_2—CH(COOM)—O—CH_2—]_2CH—O—CH(COOM)—CH_2—COOM \qquad (I)$$

(worin die Reste M, die gleich oder verschieden sein können, jeweils ein Wasserstoffatom oder ein salzbildendes Kation bedeuten), oder Mischungen davon herstellt.

4. Verfahren nach Anspruch 2, wobei man Verbindungen von Formel (II):

$$HO—CH_2—CH(OH)—CH_2—O—CH(COOM)—CH_2—COOM \qquad (II)$$

(worin die Reste M, die gleich oder verschieden sein können, jeweils ein Wasserstoffatom oder ein salzbildendes Kation bedeuten), oder Mischungen davon herstellt.

5. Verfahren nach Anspruch 2, wobei man Verbindungen von Formel (III):

$$\begin{array}{l} HO—CH_2—CH—CH_2—O—CH(COOM)—CH_2—COOM \qquad (III) \\ \qquad\qquad\quad | \\ \qquad\qquad\quad O—CH(COOM)—CH_2—COOM \end{array}$$

(worin die Reste M, die gleich oder verschieden sein können, jeweils ein Wasserstoffatom oder ein salzbildendes Kation bedeuten), oder Mischungen davon herstellt.

6. Verfahren nach Anspruch 2, wobei man Verbindungen von Formel (IV):

$$MOOC—CH_2—CH(COOM)—O—CH_2—CH(OH)—CH_2—O—CH(COOM)—CH_2—COOM \qquad (IV)$$

(worin die Reste M, die gleich oder verschieden sein können, jeweils ein Wasserstoffatom oder ein salzbildendes Kation bedeuten), oder Mischungen davon herstellt.

7. Verfahren nach Anspruch 2, wobei man Verbindungen von Formel (V):

$$HO—CH_2—CH—CH_2—OH \quad (V)$$
$$|$$
$$O—CH(COOM)—CH_2—COOM$$

(worin die Reste M, die gleich oder verschieden sein können, jeweils ein Wasserstoffatom oder ein salzbildendes Kation bedeuten), oder Mischungen davon herstellt.

8. Verfahren nach einem der Ansprüche 1 bis 7, wobei man als Quelle für Maleinsäure Maleinsäureanhydrid verwendet.

9. Verfahren nach einem der Ansprüche 1 bis 8, wobei man als Erdalkalimetallhydroxid Calciumhydroxid verwendet.

10. Verfahren nach einem der Ansprüche 1 bis 9, wobei man die Reaktion bei einem pH-Wert von über 10 und unter 14 durchführt.

11. Verfahren nach einem der Ansprüche 1 bis 10, wobei man die Reaktion bei einem pH-Wert zwischen 10,5 und 12,5 durchführt.

12. Verfahren nach einem der Ansprüche 1 bis 11, wobei man die Reaktion bei einer Temperatur von mehr als 50°C durchführt.

13. Verfahren nach einem der Ansprüche 1 bis 12, wobei man nach der Reaktion von Glyzerin mit der Quelle für Maleinsäure die Erdalkalimetallionen durch Ausfällen unter Verwendung eines löslichen Carbonatsalzes entfernt.

14. Verfahren nach einem der Ansprüche 1 bis 13, wobei man nacheinander Portionen von Maleinsäureanhydrid und von Calciumhydroxid zu einer Glyzerin enthaltenden Reaktionsmischung gibt.

15. Chemisches Mittel, das wenigstens eine Verbindung der Formel (A) (wie in Anspruch 2 angegeben), oder ein Salz davon zusammen mit wenigstens einem Trägermaterial oder einem chemisch aktiven Agens enthält.

16. Mittel nach Anspruch 15, das als chemisch aktives Agens ein oberflächenaktives Agens enthält.

17. Mittel nach Anspruch 16, wobei das oberflächenaktive Agens ausgewählt ist unter Alkylethersulfaten, Alkylbenzolsulfonaten, Alkylsulfaten, Olefinsulfonaten, Paraffinsulfonaten, Alkylpolyglycosiden, alkoxylierten Alkoholen und Mischungen von zwei oder mehreren davon.

18. Mittel nach einem der Ansprüche 15 bis 17, das als chemisch aktives Agens einen Detergensbuilder enthält, ausgewählt unter 2 - Oxa - 1,3,4 - butantricarbonsäuresalzen, 2 - Oxa - 1,1,3 - propantricarbonsäuresalzen, Zeoliten, Citratsalzen, Carbonatsalzen, Silikaten, Phosphatsalzen und Nitrilotriessigsäuresalzen.

19. Mittel nach einem der Ansprüche 15 bis 18 als flüssiges Detergensprodukt.

20. Verwendung von Verbindungen der Formel (A) (wie in Anspruch 2 angegeben) oder Salzen davon in oder als Detergensprodukt(en).

21. Verwendung von Verbindungen der Formel (A) (wie in Anspruch 2 angegeben) oder Salzen davon als Korrosionsinhibitoren.

22. Verfahren zur Herstellung von chemischen Mitteln nach einem der Ansprüche 15 bis 19, wobei man Verbindungen der Formel (A) (wie in Anspruch 2 angegeben), oder Salze davon mit einem Trägermaterial und/oder einem chemisch aktiven Agens mischt.

**Revendications pour les Etats Contractants: BE, CH, DE, FR, GB, IT, LI, NL, SE**

1. Composés de la formule A

$$R^1O—CH_2—CH(OR^2)—CH_2—OR^3, \quad (A)$$

(dans laquelle $R^1$, $R^2$ et $R^3$, qui peuvent être identiques ou différents, désignent chacun un atome d'hydrogène ou un groupe de la formule $—CH(COOH)—CH_2—COOH$, l'un au moins de $R^1$, $R^2$ et $R^3$ devant cependant être autre qu'un atome d'hydrogène) ou les sels ou mélanges de deux ou de plus de deux de ces composés et sels.

2. Composés suivant la revendication 1, de la formule I

$$[MOOC—CH_2—CH(COOM)—O—CH_2—]_2CH—O—CH(COOM)—CH_2—COOM \quad (I)$$

(dans laquelle les différents M, qui peuvent être identiques ou différents, désignent chacun un atome d'hydrogène ou un cation salifiant) et les mélanges de ceux-ci.

3. Composés suivant la revendication 1, de la formule II

$$HO—CH_2—CH(OH)—CH_2—O—CH(COOM)—CH_2—COOM \quad (II)$$

(dans laquelel les différents M, qui peuvent être identiques ou différents, désignent chacun un atome d'hydrogène ou un cation salifiant) et les mélanges de ceux-ci.

4. Composés suivant la revendication 1, de la formule III

$$\begin{array}{l} HO{-}CH_2{-}CH{-}CH_2{-}O{-}CH(COOM){-}CH_2{-}COOM \qquad (III) \\ \qquad\qquad\quad | \\ \qquad\qquad\quad O{-}CH(COOM){-}CH_2{-}COOM \end{array}$$

(dans laquelle les différents M, qui peuvent être identiques ou différents, désignent chacun un atome d'hydrogène ou un cation salifiant) et les mélanges de ceux-ci.

5. Composés suivant la revendication 1, de la formule IV

$$MOOC{-}CH_2{-}CH(COOM){-}O{-}CH_2{-}CH(OH){-}CH_2{-}O{-}CH(COOM){-}CH_2{-}COOM \qquad (IV)$$

(dans laquelle les différentes M, qui peuvent être identiques ou différents, désignent chacun un atome d'hydrogène ou un cation salifiant) et les mélanges de ceux-ci.

6. Composés suivant la revendication 1, de la formule V

$$\begin{array}{l} HO{-}CH_2{-}CH{-}CH_2{-}OH \qquad (V) \\ \qquad\qquad\quad | \\ \qquad\qquad\quad O{-}CH(COOM){-}CH_2{-}COOM \end{array}$$

(dans laquelle les différents M, qui peuvent être identiques ou différents, désignent chacun un atome d'hydrogène ou un cation salifiant) et les mélanges de ceux-ci.

7. Composés suivant l'une quelconque des revendications 1 à 6 sous forme d'acides libres.

8. Composés suivant l'une quelconque des revendications 1 à 6 sous forme de sels, dont le cation est un cation sodium, potassium, calcium, magnésium ou ammonium éventuellement substitué.

9. Mélange suivant la revendication 1 de composés de la formule I telle que définie dans la revendication 2 et de composés de la formule III telle que définie dans la revendication 4.

10. Mélange suivant la revendication 1 de composés de la formule I telle que définie dans la revendication 2 et de composés de la formule IV telle que définie dans la revendication 5.

11. Mélange suivant l'une des revendications 9 et 10, dans lequel le rapport pondéral des composés est compris entre 8:1 et 1:8.

12. Composition chimique, contenant au moins un composé de la formule A telle que définie dans l'une quelconque des revendications 1 à 11, ou un sel d'un tel composé en mélange avec au moins un matériau support ou un agent chimiquement actif.

13. Composition suivant la revendication 12, dans laquelle l'agent chimiquement actif est un surfactif ou agent tenio-actif.

14. Composition suivant la revendication 13, dans laquelle le surfactif est choisi parmi les alkyléther-sulfates, les alkyl-benzène-sulfonates, les alkylsulfates, les oléfine-sulfonates, les paraffine-sulfonates, les alkyl-polyglucosides et les alcools alcoxylés et les mélanges de deux ou de plus de deux de ces substances.

15. Composition suivant l'une quelconque des revendications 12 à 14, contenant comme agent chimiquement actif un adjuvant de détergence choisi parmi les sels de l'acide 2 - oxa - 1,3,4 - butane-tricarboxylique, les sels de l'acide 2 - oxa - 1,1,3 - propane - tricarboxylique, les zéolithes, les citrates, carbonates, silicates et phosphates et les sels de l'acide nitrilo-triacétique.

16. Composition suivant l'une quelconque des revendications 12 à 15 sous forme d'un produit détergent liquide.

17. Utilisation des composés, sels et compositions suivant l'une quelconque des revendications 1 à 16 comme détergents ou dans des produits détergents.

18. Utilisation de sels suivant l'une quelconque des revendications 1 à 11 comme inhibiteurs de la corrosion.

19. Procédé de préparation de dérivés du glycérol, qui consiste à faire réagir le glycérol, en présence d'un hydroxyde d'un métal alcalino-terreux, avec une source d'acide maléique.

20. Procédé suivant la revendication 19, dans lequel la source d'acide maléique est l'anhydride maléique.

21. Dérivés du glycérol, préparés par le procédé suivant l'une quelconque des revendications 19 et 20.

22. Procédé de préparation de compositions chimiques suivant l'une quelconque des revendications 12 à 16, qui consiste à mélanger à des composés de la formule A ou des sels de ceux-ci un matériau support ou un agent chimiquement actif tel que défini.

**Revendications pour l'Etat Contractant: AT**

1. Procédé de préparation de dérivés du glycérol, qui consiste à faire réagir le glycérol, en présence d'un hydroxyde d'un métal alcalino-terreux, avec une source d'acide maléique.

2. Procédé suivant la revendication 1, pour la préparation de composés de la formule A

$$R^1O—CH_2—CH(OR^2)—CH_2—OR^3 \qquad (A)$$

(dans laquelle $R^1$, $R^2$ et $R^3$, qui peuvent être identiques ou différents, désignent chacun un atome d'hydrogène ou un groupe de la formule —CH(COOH)—$CH_2$—COOH, l'un au moins de $R^1$, $R^2$ et $R^3$ devant cependant être autre qu'un atome d'hydrogène) ou de sels ou de mélanges de ceux-ci.

3. Procédé suivant la revendication 2 pour la préparation de composés de la formule I

$$[MOOC—CH_2—CH(COOM)—O—CH_2—]_2CH—O—CH(COOM)—CH_2—COOM \qquad (I)$$

(dans laquelle les différents M, qui peuvent être identiques ou différents, désignent chacun un atome d'hydrogène ou un cation salifiant) ou de mélanges de ceux-ci.

4. Procédé suivant la revendication 2 pour la préparation de composés de la formule II

$$HO—CH_2—CH(OH)—CH_2—O—CH(COOM)—CH_2—COOM \qquad (II)$$

(dans laquelle les différents M, qui peuvent être identiques ou différents, désignent chacun un atome d'hydrogène ou un cation salifiant) ou de mélanges de ceux-ci.

5. Procédé suivant la revendication 2 pour la préparation de composés de la formule III

HO—$CH_2$—CH—$CH_2$—O—CH(COOM)—$CH_2$—COOM (III)
|
O—CH(COOM)—$CH_2$—COOM

(dans laquelle les différents M, qui peuvent être identiques ou différents, désignent chacun un atome d'hydrogène ou un cation salifiant) ou de mélanges de ceux-ci.

6. Procédé suivant la revendication 2 pour la préparation de composés de la formule IV

$$MOOC—CH_2—CH(COOM)—O—CH_2—CH(OH)—CH_2—O—CH(COOM)—CH_2—COOM \qquad (IV)$$

(dans laquelle les différents M, qui peuvent être identiques ou différents, désignent chacun un atome d'hydrogène ou un cation salifiant) ou de mélanges de ceux-ci.

7. Procédé suivant la revendication 2 pour la préparation de composés de la formule V

HO—$CH_2$—CH—$CH_2$—OH
|
O—CH(COOM)—$CH_2$—COOM

(dans laquelle les différents M, qui peuvent être identiques ou différents, désignent chacun un atome d'hydrogène ou un cation salifiant) ou de mélanges de ceux-ci.

8. Procédé suivant l'une quelconque des revendications 1 à 7, dans lequel la source d'acide maléique est l'anhydride maléique.

9. Procédé suivant l'une quelconque des revendications 1 à 8, dans lequel l'hydroxyde de métal alcalino-terreux est l'hydroxyde de calcium.

10. Procédé suivant l'une quelconque des revendications 1 à 9, dans lequel la réaction est réalisée à un pH supérieure à 10 et inférieur à 14.

11. Procédé suivant l'une quelconque des revendications 1 à 10, dans lequel la réaction es réalisée à un pH compris entre 10,5 et 12,5.

12. Procédé suivant l'une quelconque des revendications 1 à 11, dans lequel la réaction est réalisée à une température supérieure à 50°C.

13. Procédé suivant l'une quelconque des revendications 1 à 12, dans lequel la réaction du glycérol et de la source d'acide maléique est suivie de l'élimination des ions de métal alcalino-terreux par précipitation à l'aide d'un carbonate soluble.

14. Procédé suivant l'une quelconque des revendications 1 à 13, dans lequel l'on ajoute au mélange réactionnel contenant du glycérol des quantités additionnelles d'anhydride maléique et d'hydroxyde de calcium.

15. Composition chimique, contenant au moins un composé de la formule A, telle que définie dans la revendication 2, ou un sel d'un tel composé en mélange avec au moins un matériau support ou agent chimiquement actif.

16. Composition suivant la revendication 15, dans laquelle l'agent chimiquement actif est un surfactif (ou agent tensio-actif).

17. Composition suivant la revendication 16, dans laquelle le surfactif est choisi parmi les alkyléther-sulfates, les alkyl-benzène-sulfonates, les alkyl-sulfates, les oléfine-sulfonates, les paraffine-sulfonates, les alkyl-poly-glucosides, les alcools alcoxylés et les mélanges de deux ou de plus de deux de ces composés.

18. Composition suivant l'une quelconque des revendications 15 à 17, dans laquelle l'agent chimiquement actif est un adjuvant de détergence choisi parmi les sels de l'acide 2 - oxa - 1,3,4 - butane - tricarboxylique, les sels de l'acide 2 - oxa - 1,1,3 - propane - tricarboxylique, les zéolithes, les citrates, carbonates, silicates et phosphates et les sels de l'acide nitrilo-triacétique.

19. Composition suivant l'une quelconque des revendications 15 à 18 sous forme d'un produit détergent liquide.

20. Utilisation de composés de la formule A, telle que définie dans la revendication 2, et de sels de ceux-ci en tant que détergents ou dans des compositions détergentes.

21. Utilisation de composés de la formule A, telle que définie dans la revendication 2, ou de sels de ceux-ci comme inhibiteurs de la corrosion.

22. Procédé de préparation de compositions chimiques suivant l'une quelconque des revendications 15 à 19, dans lequel on mélange des composés de la formule A, telle que définie dans la revendication 2, ou des sels de ceux-ci à un matériau support et(ou) à un agent chimiquement actif.

FIG. 1

THE SEQUESTRATION OF Ca++ BY GTS AND KNOWN BUILDERS VIA THE Ca-- ELECTRODE METHODE